# EUROPÄISCHE PATENTSCHRIFT

(11) **EP 0 938 679 B1**
(45) Veröffentlichungstag und Bekanntmachung des Hinweises auf die Patenterteilung: **24.07.2002**
(21) Anmeldenummer: 97952757.9
(22) Anmeldetag: 05.11.1997
(51) Int. Cl.: G01N 33/76, G01N 33/566, G01N 33/564

(54) **REZEPTORBINDUNGSASSAY, FÜR DEN REZEPTORBINDUNGSASSAY GEEIGNETER REKOMBINANTER FUSIONSREZEPTOR, VEKTOR ZU DESSEN HERSTELLUNG SOWIE REAGENZIENSATZ FÜR DIE DURCHFÜHRUNG DES REZEPTORBINDUNGSASSAYS**
RECEPTOR BINDING ASSAY, APPROPRIATE RECOMBINANT FUSION RECEPTOR FOR SAID ASSAY, VECTOR FOR ITS PRODUCTION AND REAGENT KIT FOR IMPLEMENTING THE RECEPTOR BINDING ASSAY
TEST DE LIAISON D'UN RECEPTEUR, RECEPTEUR DE FUSION RECOMBINE APPROPRIE POUR CE TEST, VECTEUR POUR SA PREPARATION ET JEU DE REACTIFS POUR EFFECTUER CE TEST

(30) Priorität: 06.11.1996 DE 19645729; 07.07.1997 DE 19728991
(43) Veröffentlichungstag der Anmeldung: 01.09.1999
(73) Patentinhaber: B.R.A.H.M.S Aktiengesellschaft, 16761 Hennigsdorf (DE)
(72) Erfinder: LOOS, Ulrich, D-89081 Ulm (DE); MINICH, Waldemar, B., D-89075 Ulm (DE)
(74) Vertreter: Andrae, Steffen, Dr.
(86) Internationale Anmeldenummer: EP9706121
(87) Internationale Veröffentlichungsnummer: WO9820343

(56) Entgegenhaltungen:
- EP-A- 0 433 509
- EP-A- 0 482 598
- WO-A-91/09137
- DE-C- 19 522 171
- US-A- 5 284 778
- NAGAYAMA, YUJI ET AL: "Binding domains of stimulatory and inhibitory thyrotropin (TSH) receptor autoantibodies determined with chimeric TSH-lutropin/chorionic gonadotropin receptors" J. CLIN. INVEST. (1991), 88(1), 336-40 CODEN: JCINAO;ISSN: 0021-9738, 1991, XP002062530
- PATENT ABSTRACTS OF JAPAN vol. 011, no. 193 (C-430), 20.Juni 1987 & JP 62 019087 A (KIYOSHI MIYAI;OTHERS: 02), 27.Januar 1987,

## Beschreibung

Die vorliegende Erfindung betrifft einen Rezeptorbindungsassay, insbesondere zur Bestimmung von TSH-Rezeptor-Autoantikörpern in einer biologischen Probe, unter Verwendung von rekombinanten Fusionsrezeptoren, die zur Bestimmung von TSH-Rezeptor-Autoantikörpern mit Hilfe eines derartigen Assays erforderlichen rekombinanten TSH-Fusionsrezeptoren, einen Vacciniavirus-Vektor zur Herstellung derartiger Fusionsrezeptoren und einen Reagenziensatz (Kit) zur Durchführung eines erfindungsgemäßen Rezeptorbindungsassays.

Es ist bekannt, biologisch aktive Substanzen, deren Anwesenheit und/oder Menge in einer biologischen Probe, und zwar einer flüssigen oder verflüssigten biologischen Probe, gemessen werden soll, mit Hilfe von Bestimmungsverfahren (Assays) zu bestimmen, bei denen spezifische Binder für die zu bestimmende Substanz in Kombination mit weiteren Reagenzien wie markierten Kompetitoren, Immobilisierungsreagenzien, Fällungsreagenzien und/oder Markierungsreagenzien zum Einsatz kommen. Die bekanntesten derartigen Bestimmungsverfahren sind dabei immundiagnostische Bestimmungsverfahren (Immunoassays), bei denen spezifische Bindungen vom Antigen-Antikörper-Typ genutzt werden. Auch wenn es in Einzelfällen aufgrund der Natur der zu bestimmenden oder als Kompetitoren zu verwendenden Antigenmoleküle nicht einfach sein kann, Bestimmungsverfahren zu entwikkeln, die alle Anforderungen für einen Routineeinsatz in der klinischen Praxis erfüllen, so bereitet es doch in der Regel keine grundsätzlichen Schwierigkeiten, derartige Assays so zu gestalten, daß am Ende der Bestimmung ein Reaktionsprodukt, das Markierungsanteile enthält und damit eine Information über die Anwesenheit und/oder Menge der zu bestimmenden Substanz liefert, an eine feste Phase gebunden vorliegt, z.B. an die Wände eines Teströhrchens. Insbesondere ist es in der Regel nicht besonders schwierig, als Bestandteil eines solchen Assays verwendete spezifische Binder vom Antikörpertyp ohne Beeinträchtigung ihrer für das Bestimmungsverfahren wichtigen spezifischen Bindungsfähigkeit direkt oder indirekt zu markieren und/oder zu immobilisieren.

Anders liegen die Verhältnisse im Falle von Rezeptorbindungsassays, d.h. im Falle von Bestimmungsverfahren (Assays), bei denen wenigstens einer der spezifischen Bindungspartner ein Rezeptor ist. Die Bindung von Biomolekülen von peptidischer oder proteinischer Natur, z.B. von Hormonen oder auch Autoantikörpern, an Rezeptoren ist in der Regel sehr komplexer Natur, und die Ausbildung einer spezifischen Bindung zwischen Rezeptor und Biomolekül ist sehr viel empfindlicher gegenüber strukturellen Veränderungen insbesondere des Rezeptors, als das bei einem üblichen Bindungspaar Antigen/Antikörper der Fall ist. Versuche, Rezeptoren zu immobilisieren und/oder zu markieren, führen in der Regel zu strukturellen Veränderungen, die die Funktionalität von Rezeptoren stark beeinträchtigen. Das führt dazu, daß zahlreiche Assay-Grundtypen, die bei Immunoassays unter Ausnutzung einer Antikörper/Antigen-Bindung zur Verfügung stehen, für die Durchführung von Rezeptorbindungsassays in der Praxis nicht genutzt werden können.

In dem Patent DE 43 28 070 C1 ist ein Typ eines Rezeptorbindungsassays, der nach der Coated-Tube-Technik arbeitet, beschrieben, bei dem die Schwierigkeit der Herstellung von markierten bzw. immobilisierten funktionalen Rezeptorpräparationen dadurch umgangen wird, daß man an die Festphase Bestandteile eines kompetitierenden Reaktionsystems bindet, das gewissermaßen einen "Schatten" der eigentlichen Rezeptorbindungsreaktion darstellt. Für die Schaffung von Assays für die klinische Routinediagnostik hat sich das offenbarte Verfahrensprinzip jedoch als zu kompliziert und daher wenig praktikabel erwiesen. Auf die allgemeinen Ausführungen in der genannten Patentschrift zur Problematik von Rezeptorbindungsassays im allgemeinen und von solchen zur Bestimmung von TSH-Rezeptor-Autoantikörpern im speziellen wird ergänzend ausdrücklich Bezug genommen.

Aus der EP-B-0 488 170 sind ferner zellfreie Rezeptorbindungstests bekannt, bei denen rekombinante Fusionsrezeptoren aus einem aminoterminalen Rezeptorprotein und einem Trägerprotein, insbesondere dem konstanten Teil (Fc) der schweren Kette eines Immunglobulins, eingesetzt werden, die mittels eines Antiserums oder eines monoklonalen Antikörpers an eine feste Phase gekoppelt sind. Die diskutierten Rezeptoren gehören nicht zur Klasse der hochmolekularen G-Protein gekoppelten Glykoprotein-Rezeptoren. Ferner ist eine Immobilisierung durch Bindung eines Trägerproteins, das der Fc-Teil eines Immunglobulins ist, für Rezeptorbindungsassays, mit deren Hilfe Autoantikörper bestimmt werden sollen, wenig geeignet, da die Autoantikörper selbst zu den Immunglobulinen gehören und mit dem Immobilisierungssystem wechselwirken können.

Zu den Rezeptoren, die bisher beim Versuch ihrer Immobilisierung und/oder Markierung in ihrer Funktionalität so beeinträchtigt wurden, daß sie bisher weder in immobilisierter noch in direkt markierter Form für Bestimmungsverfahren vom Rezeptorbindungsassay-Typ verwendet werden konnten, gehört insbesondere auch der TSH-Rezeptor.

Rezeptorbindungsassays, bei denen als spezifischer Binder TSH-Rezeptor-Präparationen verwendet werden, bilden den eigentlichen Schwerpunkt der vorliegenden Erfindung. Das schließt es jedoch nicht aus, daß die nachfolgend präsentierten Erkenntnisse und vorteilhaften Ergebnisse, die mit modifizierten TSH-Rezeptoren gemäß der vorliegenden Erfindung erhalten wurden, auf andere Fälle direkt übertragen werden können, insbesondere auf solche Fälle, bei denen Rezeptoren verwendet werden, die dem gleichen Substanztyp angehören wie der TSH-Rezeptor.

Der TSH-Rezeptor ist ein in der Schilddrüsenmembran lokalisierter Rezeptor, an den das von der Hypophyse ausgeschüttete Hormon TSH (Thyroid-stimulierendes Hormon oder Thyreotropin) bindet und dadurch die Ausschüttung der eigentlichen Schilddrüsenhormone, insbesondere des Thyroxins, auslöst. Der TSH-Rezeptor gehört zur Rezeptor-Familie der G-Protein-gekoppelten Glykoprotein-Rezeptoren mit einer großen aminoterminalen extrazellulären Domäne, zu der auch der LH/CG- und der FSH-Rezeptor gehören. Eine Aufklärung der chemischen Struktur des TSH-Rezeptors, d.h. der Sequenz der für ihn codierenden DNA sowie der daraus ableitbaren Aminosäuresequenz des Rezeptors selbst, gelang Ende 1989 (vgl. Libert F. et al., Biochem. Biophys. Res. Commun. 165: 1250-1255; Nagayama Y. et al., Biochem. Biophys. Res. Commun. 165: 1184-1190; vgl. auch EP-A-0433509 bzw. WO-A-91/09121; sowie WO-A-91/09137; WO-A-91/10735 und WO-A-91/03483; ferner Yuji Nagayama & Basil Rapoport, in: Molecular Endocrinology, Vol. 6 No. 2, S. 145-156 und die darin zitierte Literatur). Mit der Verfügbarkeit der für den humanen TSH-Rezeptor codierenden cDNA gelang die Expression des rekombinanten humanen TSH-Rezeptors in einer Vielzahl verschiedener Expressionssysteme. Dabei zeigte es sich, daß ein funktionaler humaner TSH-Rezeptor voller Länge anscheinend nur in Säugetierzellen exprimiert werden kann, und zwar sowohl transient als auch in Form stabiler Zelllinien (vgl. z.B. Loos, U. et al. in: Eur. J. Biochem. 232, S. 62-65 (1995); oder Matsuba T. et al., J. Biochem. 118, S. 265-270 (1995) und darin zitierte Literatur).

Gegen den TSH-Rezeptor können Autoantikörper gebildet werden, die für verschiedene Autoimmunerkrankungen verantwortlich sind, wobei sogenannte stimulierende Autoantikörper, die zu einer als Morbus Basedow (englisch: Graves' disease) bekannten Schilddrüsenüberfunktion führen, für die klinische Diagnostik besonders wichtig sind. Ein zur Bestimmung derartiger Autoantikörper im Handel befindlicher bekannter Assay ist der TRAK-Assay® der Firma B.R.A.H.M.S Diagnostica GmbH.

Zur Bestimmung von TSH-Rezeptor-Autoantikörpern wird nach dem herkömmlichen Verfahren so vorgegangen, daß man die zu bestimmenden Autoantikörper aus einer Serumprobe in flüssiger Phase mit einem radioaktiv markierten bovinen TSH-Kompetitor um die Bindungsstellen eines Detergens-solubilisierten porcinen TSH-Rezeptors konkurrieren läßt (vgl. Southgate, K. et al., Clin. Endocrinol. (Oxford) 20, 539-541 (1984); Matsuba T. et al., a.a.O.; Produktinformation zum TRAK-Assay® der Firma B.R.A.H.M.S Diagnostica GmbH). Um das an die Rezeptor-Präparation gebundene markierte TSH zu bestimmen, wird nach Abschluß der Inkubation der TSH-Rezeptor mit einem Fällungsreagens und einem anschließenden Zentrifugierschritt von der flüssigen Phase abgetrennt. Die Bestimmung des Rezeptor-gebundenen markierten TSH erfolgt durch Messung der im Sediment gebundenen Radioaktivität.

Durch die Verfügbarkeit von rekombinanten humanen TSH-Rezeptor-Präparationen hat sich an diesem Assay-Design nichts geändert. Soweit die in den verschiedenen Expressionssystemen erhaltenen rekombinanten humanen TSH-Rezeptoren überhaupt funktionale Rezeptoren waren, wurden die daraus gewonnenen TSH-Rezeptor-Präparationen in ähnlicher Weise in solubilisierter Form eingesetzt wie eine herkömmliche porcine TSH-Rezeptor-Präparation. Außerdem wurde zu wissenschaftlichen Zwecken die Funktionalität der exprimierten Rezeptoren auch an den ganzen transformierten Zellen gemessen.

Die Freisetzung der rekombinanten humanen TSH-Rezeptoren aus den Wirtszellen, z.B. CHO- oder COS-Zellen, mit Hilfe von Detergenzien erwies sich aufgrund der Wachstumseigenschaften derartiger Zellen als schwierig und war von einer teilweisen proteolytischen Spaltung des rekombinanten Rezeptors begleitet, und die erhaltenen Präparationen waren ebensowenig ohne Funktionalitätsverlust immobilisierbar oder markierbar wie die bisherigen, aus biologischem Material gewonnenen TSH-Rezeptor-Präparationen, so daß durch die Verwendung rekombinanter TSH-Rezeptor-Präparationen keine Änderungen des traditionellen Bestimmungsverfahrens ermöglicht wurden. Die Notwendigkeit, bei allen bisher bekannten Verfahren einen Fällungs- und Zentrifugationsschritt durchzuführen, belastet jedoch die Routinebestimmung von TSH-Rezeptor-Autoantikörpern im klinischen Laboralltag, und außerdem ist sie ein Hindernis für eine Automatisierung derartiger Bestimmungen, z.B. unter Verwendung von an sich bekannten automatischen Meßsystemen.

Im Rahmen der vorliegenden Anmeldung wird unter einem "funktionalen Rezeptor" ein Rezeptor verstanden, der die für die Durchführung eines Rezeptorbindungsassays erforderliche Funktionalität aufweist, d.h. die Eigenschaft, sowohl den Kompetitor, in der Regel TSH, als auch die gesuchten Autoantikörper zu binden. Die Fähigkeit zur cAMP-Stimulation ist demgegenüber ohne größere Bedeutung. Wenn ferner davon gesprochen wird, daß die Funktionalität des TSH-Rezeptors durch einen zusätzlichen Peptidrest und/oder durch Immobilisierung bzw. Markierung nicht beeinträchtigt ist, bedeutet das nicht, daß Eigenschaften wie z.B. Bindungsaffinitäten in jeder Hinsicht, insbesondere in quantitativer Hinsicht, mit den entsprechenden Eigenschaften von Vergleichsrezeptoren natürlichen oder rekombinanten Ursprungs identisch sein müssen. Abweichungen bei Eigenschaften, die sich nicht entscheidend auf das für die Praxis wichtige Verhalten der Rezeptorpräparation in einem der nachfolgend geschilderten Rezeptorbindungsassay auswirken, sind nicht als "Beeinträchtigung" anzusehen. Ein funktionaler Rezeptor kann die vollständige Aminosäuresequenz eines natürlich vorkommenden Rezeptors enthalten, eine funktionale Teilsequenz davon oder eine Sequenz oder Teilsequenz, bei der ein Teil der natürlich vorkommenden Aminosäuren durch andere ersetzt oder weggelassen ist oder diese durch Einschübe getrennt sind, wenn dadurch die wie oben definierte Funktionalität nicht beeinträchtigt wird.

Es ist eine Aufgabe der vorliegenden Erfindung, einen Rezeptorbindungsassay, insbesondere einen Rezeptorbindungsassay zur Bestimmung von TSH-Rezeptor-Autoantikörpern, zu schaffen, bei dem mit einem funktionalen Rezeptor, insbesondere TSH-Rezeptor, gearbeitet werden kann, der immobilisierbar und/oder markierbar ist, so daß Verfahrens-Grundtypen, bei denen mit immobilisierten oder markierten spezifischen Bindern gearbeitet wird, auch für Rezeptorbindungsassays zur Anwendung gelangen können.

Es ist eine weitere Aufgabe der vorliegenden Erfindung, die dafür erforderlichen funktionalen Rezeptorpräparationen und die Mittel zu deren Herstellung zu schaffen.

Schließlich ist es auch Aufgabe der vorliegenden Erfindung, Reagenziensätze (Kits) zu schaffen, die die für Rezeptorbindungsassays neuartigen Bestimmungsverfahren verkörpern, insbesondere solche, bei denen die Coated Tube-Technik bzw. Mikrotiterplatten-Technik für den Bereich der Rezeptorbindungsassays zur Anwendung kommt.

Diese Aufgaben werden bei einem Rezeptorbindungsassay zur Bestimmung von TSH-Rezeptor-Autoantikörpern in einer biologischen Probe, insbesondere einem Humanserum, und zwar entsprechend einer allgemeinen Definition derartiger Rezeptorbindungsassays im weitesten Sinne gemäß Oberbegriff von Patentanspruch 1, dadurch gelöst, daß man als TSH-Rezeptor-Präparation eine Präparatiön eines rekombinanten TSH-Fusionsrezeptors, insbesondere eines rekombinanten humanen TSH-Fusionsrezeptors, verwendet, der gegenüber dem entsprechenden natürlich vorkommenden funktionalen TSH-Rezeptor, insbesondere gegenüber dem natürlich vorkommenden funktionalen TSH-Rezeptor voller Länge oder einem funktionalen Fragment davon, um einen endständigen oder als Einschub vorhandenen Peptidrest wie in Anspruch 1 definiert verlängert ist, wobei dieser Peptidrest (i) eine Markierung aufweist oder selektiv markierbar ist und/oder (ii) durch Bindung an einen immobilisierten selektiven Bindungspartner immobilisiert oder immobilisierbar ist, ohne daß die Funktionalität des TSH-Rezeptors beeinträchtigt ist.

Vorteilhafte Ausgestaltungen eines derartigen Rezeptorbindungsassays sind den Unteransprüchen 2 bis 11 zu entnehmen bzw. ergeben sich für den Fachmann aus den nachfolgenden Erläuterungen.

Die Erfindung betrifft ferner für derartige erfindungsgemäße Rezeptorbindungsassays erforderliche rekombinante TSH-Fusionsrezeptoren gemäß Anspruch 12 bzw. den Unteransprüchen 13 bis 15 und einen für die Herstellung eines solchen rekombinanten TSH-Fusionsrezeptors in Warmblüterzellen geeigneten Vacciniavirus-Vektor gemäß den Anspruch 16.

Ferner betrifft die vorliegende Erfindung auch einen Reagenziensatz (Kit) gemäß der Ansprüche 17 und 18, der als einen Bestandteil eine Festphase, insbesondere Coated Tubes, mit einem daran immobilisierten funktionalen Fusionsrezeptor enthält.

Die Erfindung beruht auf der Erkenntnis, daß es möglich ist, ohne Funktionalitätsverlust am C-Terminus um einen Peptidrest verlängerte funktionale Rezeptoren vom Typ des TSH-Rezeptors herzustellen und daß derartige Fusionsrezeptoren ohne Funktionalitätsverlust an spezifische Binder für den Peptidrest gebunden werden können, die immobilisiert oder immobilisierbar oder markiert oder markierbar sind.

Die im Rahmen der vorliegenden Anmeldung als "Peptidrest" bezeichnete Aminosäuresequenz, die einen natürlich vorkommenden funktionalen Rezeptor, insbesondere einen Rezeptor voller Länge oder ein funktionales Fragment davon, z.B. den Rezeptor ohne einen Teil seines C-Terminus, in seiner Sequenz oder insbesondere an seinem C-Terminus verlängert, ist vorranging dadurch gekennzeichnet, daß zu dieser Sequenz ein selektiver Bindungspartner verfügbar ist, der an die genannte Sequenz bindet, ohne einerseits die Rezeptorfunktionalität zu beeinträchtigen und ohne andererseits mit den restlichen Komponenten eines Rezeptorbindungsassays, d.h. im Falle eines Rezeptorbindungsassays zur Bestimmung von TSH-Rezeptor-Autoantikörpern mit den zu bestimmenden Autoantikörpern und/oder dem Kompetitor, z.B. ¹²⁵TSH, störende Wechselwirkungen einzugehen.

"Peptidrest" ist dabei in einem weiten Sinne ohne Einschränkung auf kurzkettige typische "Peptide" zu verstehen und umfaßt auch Aminosäureketten mit Peptidbindung, die dem Bereich der Oligopeptide oder Polypeptide bzw. sogar Proteine zugeordnet werden können. So soll die Definition z.B. noch einen Avidinrest (512 Aminosäuren, Molekulargewicht 66.000) und einen Streptavidinrest (Molekulargewicht ca. 60.000) umfassen, die selektive sehr feste Bindungen zu Biotin als selektiven Bindungspartner eingehen, d.h. Peptide mit bis zu etwa 600 Aminosäureresten. Die bevorzugten Peptidreste sind jedoch kürzer, wobei diese Peptidreste, gegebenenfalls neben zusätzlichen, auch als Spacer zum eigentlichen Rezeptormolekül dienenden Aminosäuresequenzen, das sogenannte FLAG-Epitop und/oder einen Peptidrest aus sechs Histidinresten (6His-Rest) umfassen können. Das sogenannte FLAG-Epitop ist ein Markerpeptid der Struktur N-Asp Tyr Lys Asp Asp Asp Asp Lys-C. Ein für die Expression dieses Markerpeptids verwendbarer Expressionsvektor sowie monoklonale Antikörper, die das genannte FLAG-Epitop erkennen, sind kommerziell erhältlich (für Deutschland von der Firma Integra Biosciences GmbH). Auf das Prospektmaterial zum IBI-FLAG®-System wird ergänzend bezug genommen.

Der als weiterer Rest erwähnte 6His-Peptidrest (auch als "6His affinity tag" bekannt) ist ebenfalls Teil eines bekannten kommerziellen Peptid-Marker- und Reinigungssystems. Der 6His-Peptidrest geht eine sehr feste Komplexbindung mit Metallatomen, insbesondere Nickelatomen, ein, wobei dann, wenn diese Nickelatome durch Chelatisierung fest an ein Trägerharz gebunden sind, eine sehr feste Bindung eines Proteins oder Peptids mit dem 6His-Peptidrest an das entsprechende Trägerharz erhalten wird. Ein bevorzugtes Trägerharz ist dabei das sogenannte Ni-NTA-Harz, das (für Deutschland) vertrieben wird von der Qiagen GmbH und dessen Struktur und Verwendung beschrieben wird in der Patentschrift EP-B-0 253 303. NTA steht dabei für ein an eine Trägerharzmatrix bindbares Nitrilotriessigsäurederivat. Die Verwendung von Metallchelatharzen in der Immundiagnostik zur Herstellung von Festphasen wie z.B. beschichteten Teströhrchen (Coated Tubes) gehört nicht zu den üblichen Verwendungen derartiger Harze.

Ein weiterer bevorzugter immobilisierbarer und/oder markierbarer Peptidrest für die erfindungsgemäßen TSH-Fusionsrezeptoren ist ein Peptidrest, der eine Aminosäuresequenz, z.B eine Teilsequenz eines Biotin-Enzyms, umfaßt, die posttranslational biotinylierbar ist, so daß die in einem geeigneten Expressionssystem hergestellten TSH-Fusionsrezeptoren direkt in biotinylierter Form erhalten werden können. Eine bevorzugte derartige Aminosäuresequenz eines Biotin-Enzyms ist die C-terminale Domäne einer Biotin-Carboxyl-Carrier-Protein-Untereinheit (BCCP-Untereinheit) eines Biotin-Enzyms, insbesondere die 87 Aminosäuren umfassende C-terminale Domäne der Biotin-Carboxyl-Carrier-Protein-Untereinheit einer prokaryontischen Acetyl-CoA-Carboxylase, insbesondere derjenigen von E. coli. Es zeigte sich, daß die genannte BCCP-Untereinheit postranslational mit hoher Ausbeute biotinyliert wird, und zwar nicht nur in Prokaryontenzellen, sondern auch in Wannblüterzellen wie z.B. HeLa-Zellen, was nicht unbedingt erwartet werden konnte. Es können auf diese Weise direkt biotinylierte funktionale TSH-Fusionsrezeptoren erhalten werden.

Der biotinylierte TSH-Fusionsrezeptor ermöglicht eine spezifische, sensitive und leichte direkte Detektion durch seine Markierung mit radioaktivem Biotin oder eine indirekte Detektion durch Umsetzung mit Avidin oder Streptavidin, die ein detektierbares Label enthalten, z.B. eine radioaktive Markierung oder einen Teil eines enzymatischen und Chemiluminiszenz-Nachweissystems oder andere geeignete Fusionsprodukte davon.

Wie nachfolgend und insbesondere im experimentellen Teil beschrieben ist, zeigte es sich, daß es in einem geeigneten Expressionssystem möglich ist, einen rekombinanten TSH-Rezeptor herzustellen, der an seinem C-Terminus um einen Peptidrest, im speziellen Falle ein FLAG-Epitop und einen endständigen 6His-Rest oder einen biotinylierten Peptidrest, verlängert ist, ohne daß durch die genannte endständige Verlängerung des TSH-Rezeptors zu einem TSH-Fusionsrezeptor seine Funktionalität beeinträchtigt wird.

Als Expressionssystem hat sich ein Expressionssystem als geeignet erwiesen, bei dem rekombinante Vacciniaviren in Warmblüterzellen zur Expression des TSH-Fusionsrezeptors verwendet werden. Die Verwendung von Vacciniaviren als Vektoren ist lange bekannt (vgl. z.B. J.D. Watson et al., Rekombinierte DNA, 2. Auflage, Spektrum Akademischer Verlag GmbH, S. 206; D.R. Glick et al., Molekulare Biotechnologie, Spektrum Akademischer Verlag GmbH, S. 234f.; Kieny M.P. et al., Nature, Vol. 312, S. 163-166, 1984 ; Moss B., Science 252, S.1662-1667, 1991). Die Einführung von DNA-Sequenzen, die für ein gewünschtes Peptid oder Protein kodieren, in das Genom des Vacciniavirus mittels geeigneter Plasmide ist ebenfalls grundsätzlich bekannt. Im Rahmen der vorliegenden Erfindung wurde ein Vacciniavirus-Vektor jedoch erstmals dazu verwendet, ein Protein aus der Familie der G-Protein-gekoppelten Glycosidrezeptoren mit einer großen N-terminalen extrazellulären Domäne in tierischen Zellen zu exprimieren. Als tierische Zellen wurden HeLa-Zellen verwendet, die große Mengen des humanen TSH-Fusionsrezeptors erzeugten. HeLa-Zellen weisen gegenüber den in der Literatur beschriebenen COS7-Zellen oder CHO-Zellen den Vorteil auf, daß sie schnell in hoher Dichte sowohl in einer Trägerkultur als auch in einer Suspensionskultur gezüchtet werden können.

Die im experimentellen Teil näher beschriebene Herstellung des rekombinanten TSH-Fusionsrezeptors läßt sich kurz wie folgt zusammenfassen: Die TSH-Rezeptor-cDNA wurde in die DNA des Genoms des Vacciniavirus in Analogie zu einer vorbekannten Arbeitsweise (Kieny, M.P. et al., Nature, 312, S. 163-166 (1984)) eingeführt. Die Fremdgene benötigen für eine wirksame Transkription virale Promotoren. Das verwendete Rekombinations-Plasmid p7.5K131 enthält den Early/Late-Promotor 7.5K des Vacciniavirus, flankiert von den Vacciniavirus-Thymidinkinase(TK)-Sequenzen, um die Rekombination in den TK-Ort des viralen Genoms zu dirigieren. Das Rekombinations-Plasmid wurde so konstruiert, daß in den erhaltenen rekombinanten Viren das TSH-Rezeptor-cDNA-Segment strangabwärts von der 5'-untranslatierten Leadersequenz des Encephalomyocarditis-Virus (EMCV) flankiert ist. Diese Sequenz stammte aus dem pTMl-Vektor und erhöht die Wirksamkeit der mRNA-Translation in Zellen, die mit dem Vacciniavirus infiziert waren (Moss, B. et al., Nature, 348, S.19-92, (1990)). Rekombinante Virusklone mit der EMCV-Leader-Sequenz erzeugten sehr viel mehr TSH-Rezeptor als solche ohne diese Sequenz (Daten nicht gezeigt). Der rekombinante TSH-Rezeptor enthielt ferner das FLAG-Oktapeptid-Epitop, das von kommerziell erhältlichen monoklonalen Antikörpern erkannt wird, sowie ein 6His-Peptid an seinem Carboxyl-Terminus. Gemäß einer anderen bevorzugten Ausführungsform enthielt der rekombinante TSH-Fusionsrezeptor eine biotinylierte Peptidsequenz, und zwar die 87 Aminosäuren umfassende C-terminale Domäne der Biotin-Carboxyl-Carrier-Protein-Untereinheit der Acetyl-CoA-Carboxylase von E.coli.

Das obengenannte Rekombinations-Plasmid p7.5K131 ist unter der Bezeichnung pAvB2 beschrieben in einer Doktorarbeit (Ruprecht-Karls-Universität Heidelberg) von Herrn Albrecht von Brunn aus dem Jahre 1989 mit dem Titel "Das Oberflächenantigen des Hepatitis B Virus als Trägersystem von Epitopen eines Merozoiten-Oberflächenmoleküls des Malaria-Erregers Plasmodium falciparum - eine neue Möglichkeit der Impfstoffentwicklung?". Die Verwendung des konkreten Plasmids ist nicht kritisch, und es kann stattdessen auch mit einem anderen Vektor gearbeitet werden, der ein Thymidinkinase-Gen und den sog. 7.5K-Promotor enthält, z.B. mit dem Vektor pZVneo5529, der beschrieben ist in C. Bouvier et al., European Journal of Pharmacology, Molecular Pharmacology Section 290 (1995), S.11-17.

Rekombinante Virus-Klone wurden durch Western-Blot-Analyse des TSH-Rezeptors, der in infizierten HeLa-Zellen exprimiert wurde, identifiziert.

Der mit Hilfe des Vacciniavirus-Vektors in HeLa-Zellen hergestellte TSH-Fusionsrezeptor erwies sich als vollständig funktional im Hinblick auf die TSH-Bindung. Infizierte HeLa-Zellen wiesen hochaffine TSH-Bindungsstellen auf, wobei die Bindungsaffinität derjenigen des nicht-rekombinanten TSH-Rezeptors in humanen Schilddrüsenzellen sowie in FRTL5-Zellen bemerkenswert ähnlich ist. Es erwies sich ferner, daß der TSH-Rezeptor aus infizierten HeLa-Zellen durch das nicht-ionische Detergens Triton® X100 in wirksamer Weise extrahiert werden kann. Die durch Extraktion erhaltene Fusionsrezeptor-Präparation ist sowohl im Hinblick auf die Bindung von TSH als auch von Morbus Basedow-Autoantikörpern aktiv. Die Verlängerung des natürlich vorkommenden funktionalen TSH-Rezeptors um einen Peptidrest, im vorliegenden Falle das FLAG-Epitop und den 6His-Peptidrest oder die 87 Aminosäuren umfassende C-terminale Domäne der Biotin-Carboxyl-Carrier-Protein-Untereinheit der Acetyl-CoA-Carboxylase von E.coli, beeinträchtigte weder die Fähigkeit zur Bindung des TSH-Hormons noch zur Bindung von Autoantikörpern, und der TSH-Fusionsrezeptor war auch im Hinblick auf die TSH-stimulierte cAMP-Produktion voll funktionsfähig.

Mit dem rekombinanten Vacciniavirus infizierte HeLa-Zellen produzierten erhebliche Mengen an humanem TSH-Fusionsrezeptor, und zwar in einer Größenordnung von bis zu 150.000 funktionellen Rezeptormolekülen pro Zelle. Dieser Wert ist höher als die für die meisten vorbekannten Expressionssysteme beschriebenen Werte und vergleichbar mit dem, der von Matsuba et al., J.Biochim. 118, S. 265-270 (1995) beschrieben wird.

Die in den folgenden Beispielen an den TSH-Rezeptor angefügten Peptidreste können als Modelle für markierbare und immobilisierbare Peptidreste angesehen werden, ohne daß die Erfindung auf die genannten konkreten Reste beschränkt sein soll. Über das FLAG-Epitop kann der rekombinante TSH-Fusionsrezeptor unter Verwendung von spezifischen verfügbaren monoklonalen Antikörpern immobilisiert, jedoch auch spezifisch markiert werden. Über den 6His-Peptidrest kann der TSH-Fusionsrezeptor ebenfalls ohne Verlust seiner Funktionalität an einer Festphase immobilisiert werden und steht damit für Rezeptorbindungsassays zu Verfügung, die Verfahrensvarianten verwirklichen, die für solche Assays bisher nicht genutzt werden konnten. Über das nach der Biotinylierung kovalent an den exprimierten BCCP-Peptidteil gebundene Biotinmolekül ist ebenfalls eine Markierung und/oder Immobilisierung des entsprechenden Fusionsrezeptors möglich.

So ergeben sich aufgrund der verfügbarkeit eines immobilisierbaren und/oder markierbaren funktionalen TSH-Rezeptors sowie unter Berücksichtigung der Konkurrenz und damit bis zu einem gewissen Grad auch gegenseitigen Ersetzbarkeit. von.TSH.und TSH-Rezeptor-Antikörpern beispielsweise die folgenden Verwirklichungsmöglichkeiten für einen erfindungsgemäßen Rezeptorbindungsassay.
1. Der Fusionsrezeptor wird über seinen Peptidrest und einen spezifischen immobilisierten Bindungspartner an einer Festphase, z.B. den Wänden von Coated Tubes, immobilisiert, und die Bestimmung von TSH-Rezeptor-Autoantikörpern erfolgt analog zum vorbekannten Verfahren durch deren Konkurrenz mit markiertem TSH um die Bindungsstellen des Rezeptors.
2. Dieses Reaktionsschema kann jedoch auch umgekehrt werden, indem man TSH an einer Festphase immobilisiert und das immobilisierte TSH mit den zu bestimmenden Autoantikörpern um einen über den Peptidrest markierten TSH-Fusionsrezeptor konkurrieren läßt. Die Markierung kann beispielsweise mit Hilfe eines markierten monoklonalen Antikörpers erfolgen, der spezifisch an den Peptidrest bindet. Die Menge der über das immobilisierte TSH an die Festphase gebundenen Markierung ist hierbei umgekehrt proportional zur Konzentration der Autoantikörper in der Probe.
3. Wie oben unter 1. wird mit einem immobilisierten TSH-Rezeptor gearbeitet, wobei man als Kompetitor jedoch nicht markiertes TSH verwendet, sondern markierte monoklonale oder polyklonale TSH-Rezeptor-Antikörper, die mit den zu bestimmenden Autoantikörpern um die Bindungsstellen des immobilisierten Rezeptors konkurrieren.
4. Analog zu der unter 2. beschriebenen Verfahrensvariante kann anstelle von TSH auch ein TSH-Rezeptor-Antikörper immobilisiert werden.
5. Ein an einer Festphase immobilisierter TSH-Rezeptor kann dazu verwendet werden, die in einer Probe vorhandenen TSH-Rezeptor-Autoantikörper vollständig zu binden, die dann anschließend durch ein geeignetes Markierungsreagens, das auf Antikörper anspricht, z.B. ein Antiserum oder Protein A in markierter Form, markiert werden können.
6. Eine weitere Verfahrensvariante stellt die Umkehrung der unter 5. beschriebenen Verfahrensvariante dar, indem zuerst mit Hilfe geeigneter spezifischer oder auch unspezifischer Binder die Autoantikörper in der Probe an eine Festphase gebunden und anschließend mit Hilfe eines markierten TSH-Rezeptors markiert werden.
7. Ferner kann das Bestimmungsverfahren auch zur Bestimmung spezieller, zwei Rezeptor-bindende Epitope aufweisender Autoantikörper dienen, indem man mit einem an einer Festphase immobilisierten Rezeptor und einem löslichen markierten Rezeptor arbeitet und die Menge an Markierung an der Festphase bestimmt, die auf eine Bindung der zu bestimmenden Autoantikörper sowohl durch den immobilisierten als auch den markierten Rezeptor zurückgeht.

Weitere an sich bekannte Assaydesigns, z.B. solche mit einer stufenweisen Inkubation und einer Differenzmessung, sind dem Fachmann ohne weiteres bekannt.

Als Antikörper können bei diesen Assaydesigns geeignete monoklonale und polyklonale Antikörper, insbesondere auch humane monoklonale und humane polyklonale Antikörper, sowie rekombinante Antikörperkonstrukte, z.B. sogenannte Single-Chain-Antikörper, verwendet werden. Es ist ferner möglich, auch geeignete Antikörperfragmente einzusetzen, insbesondere auch zur Rezeptormarkierung.

Die Verfügbarkeit der erfindungsgemäßen funktionalen Fusionsrezeptoren macht es auch möglich, neue definierte monoklonale Antikörper gegen den rekombinanten funktionalen TSH-Fusionsrezeptor zu erzeugen, die gegen Teile der Rezeptorsequenz und angrenzende Teile des angehängten Peptidrests, z.B. das FLAG-Epitop und/oder einen Teil des 6His-Peptids, gerichtet sind, und die aufgrund ihrer Spezifität weitere neue Anwendungsmöglichkeiten und Vorteile für die Assaygestaltung erschließen. Weitere Informationen zur Durchführung und zu den Vorteilen der vorliegenden Erfindung ergeben sich für den Fachmann aus dem nachfolgenden experimentellen Teil unter Bezugnahme auf die Figuren. In den Figuren zeigen:
- Figur 1:: den zeitlichen Verlauf der TSH-Rezeptor-Expression in HeLa-Zellen. In Mikrotiterplatten mit 24 Vertiefungen wurden HeLa-Monolayer-Zellen mit einer Plaque-bildenden Einheit des rekombinanten Vacciniavirus pro Zelle infiziert. 0, 2, 4, 6, 8, 14 und 24 h nach der Infektion wurden die Zellen entnommen, mittels SDS-Page aufgetrennt, und der TSH-Rezeptor wurde durch Western-Blot-Analyse nachgewiesen. Die Molekülgrößenmarker sind in kDa angegeben.
- Figur 2:: die Bindung von ¹²⁵I-TSH an Extrakte von infizierten HeLa-Zellen als Funktion der Infektionszeit. Die Bestimmung erfolgte unter Verwendung der Bestandteile des TRAK-Assays® in einem Gesamtvolumen von 100µl gemäß Gebrauchsanweisung.
- Figur 3:: die Verteilung des rekombinanten TSH-Fusionsrezeptors auf unterschiedliche Zellfraktionen, die aus infizierten HeLa-Zellen erhalten wurden. In der Darstellung stellt Bande 1 einen Markerextrakt von HeLa-Zellen dar, Bande 2 repräsentiert die wasserlösliche Cytoplasmafraktion, Bande 3 den unter Verwendung von Triton® X100 erhaltenen Membranextrakt, Bande 4 das verbleibende, mit Triton® X100 nicht extrahierbare unlösliche Protein. Die Molekülgrößenmarker sind wiederum in kDa angegeben.
- Figur 4:: eine Scatchard-Darstellung der ¹²⁵I-TSH-Bindung an mit dem rekombinanten Vacciniavirus infizierten HeLa-Zellen.
- Figur 5:: die Analyse von Autoantikörpern in unfraktionierten Seren von Morbus Basedow-Patienten im Vergleich mit Kontrollseren von Normalpatienten. Die Wirkung von sechs Kontrollseren und 13 Seren von Morbus Basedow-Patienten auf die Bindung von ¹²⁵I-TSH an den rekombinanten TSH-Rezeptor im Extrakt aus infizierten HeLa-Zellen wurde im Vergleich mit dem porcinen TSH-Rezeptor (aus dem TRAK-Assay® Kit) untersucht. Die Ergebnisse sind angegeben als Prozentsatz der Gesamtbindung, wobei eine 100%ige Bindung für die ¹²⁵I-TSH-Bindung in Gegenwart eines Nullstandard-Serums steht. Die Ergebnisse sind als Durchschnittswerte von Doppelbestimmungen angegeben.
- Figur 6:: eine Standardkurve, wie sie unter Verwendung des an Ni-NTA-Agarose gekoppelten TSH-Fusionsrezeptors und TRAK-Assay® Standards erhalten wird.
- Figur 7:: die Meßergebnisse für die Bestimmung der TSHr-Autoantikörper-Titer von Morbus Basedow-Patienten und einer Kontrollgruppe unter Verwendung von an Ni-NTA-Agarose gekoppeltem rekombinantem TSH-Fusionsrezeptor; und
- Figur 8:: die für Normalpatienten und Morbus Basedow-Patienten erhaltenen Meßergebnisse in einer vergleichenden graphischen Gegenüberstellung.

### Experimenteller Teil

### Materialien

Bovines¹²⁵I-TSH(56 µCi/µg), unmarkiertes TSH sowie der TRAK-Assay®-Kit wurden von der Fa. B.R.A.H.M.S Diagnostica GmbH zur Verfügung gestellt. Der pTM1-Vektor wurde von den Doktoren R.E. Rhoads und B. Joshi (Louisiana State University Medical Center, Shreveport, USA) bezogen, der p7.5K131-Rekombinationsvektor wurde von Dr.T.Mertens (Universität Ulm, Deutschland) zur Verfügung gestellt. Der BIOTRAK® cAMP [¹²⁵I]-Assay-Kit und der ECL®-Western-Blot-Nachweis-Kit wurden von der Fa. Amersham bezogen. Die monoklonalen Mäuse-Antikörper NCL-TSHR gegen die extrazelluläre Domäne des TSH-Rezeptors wurden von der Fa. Novocastra Laboratories Ltd. bezogen. Proteaseninhibitorcocktail-Tabletten wurden von der Fa. Boehringer Mannheim bezogen. Die DNA-Primer P1 bis P5 wurden von der Fa. Eurogentec bezogen. Sie wiesen die folgenden Nucleotidsequenzen auf:

P1:    5'- GCT GCT GTC ATG TGA AGA TCT ACA TCA CAG TCC GAA ATC
           CGC AGT ACA ACC CAG G - 3'

P2: 5' - CGC GGA TCC TTA CTT GTC ATC GTC GTC CTT GTA GTC CAA
AAC CGT TTG CAT ATA C - 3'

P3: 5′ - CGC GGA TCC TTA GTG ATG GTG ATG GTG ATG CTT GTC ATC
GTC GTC CTT G - 3′

P4: 5' - GTA ACC CGA GTC CCG TGG ATC ATG AGG CCG G - 3'

P5: 5' - GGG CAG TGA CAC TGG TTT GAG ACA CGT CCA G - 3'

Das pSK1-Plasmid, das eine Sequenz für die 87 Aminosäuren umfassende C-terminale Domäne der Biotin-Carboxyl-Carrier-Protein (BCCP) Untereinheit der Acetyl-CoA-Carboxylase von E.coli enthielt, war ein Geschenk von Dr. J. Gelles (Dept. of Biochemistry, Brandeis University, 415 South Street, Waltham, MA 02254; vgl. Lit. Berliner et al. in J. Biol. Chem. 269: Seite 8610-8615, 1994). Die Primer P10 und P11 wiesen die folgenden Nucleotidsequenzen auf: P10: 5' - GTC GGT TAC CAT GGA AGC GCC AGC AGC AG - 3 und P11: 5' - CGC GGA TCC TTA TTC GAT AAC AAC AAG CGG TTC - 3'. Sie wurden ebenfalls von der Firma Eurogentec bezogen.

Soweit für einzelne Reagenzien keine Bezugsquellen angegeben werden, wurden allgemein frei erhältliche Handelsprodukte verwendet, bei denen die Bezugsquellen für die Nacharbeitbarkeit keine Bedeutung haben. Soweit ferner nachfolgend für einzelne Reaktionsschritte keine speziellen Reaktionsbedingungen angegeben sind, wurde unter den fachnotorisch bekannten Bedingungen gearbeitet, z.B. bei den für die jeweiligen Restriktionsenzyme angebenen optimalen Temperaturen und Reaktionszeiten.

### Zellkultur

HeLa-Zellen wurden in Eagle's-Medium (modifiziert nach Dulbecco) gezüchtet, das mit 10% fötalem Kälberserum ergänzt war, das 50 U/ml Penicillin und 50 µg/ml Streptomycin enthielt. Die Zellen wurden bei 37°C unter einer 5%igen CO₂-Atmosphäre kultiviert.

### A. Herstellung und Prüfung eines FLAG-6HIS-TSH-Fusionsrezeptors

### Herstellung eines um das FLAG-Octapeptid-Epitop sowie eine 6His-Peptidsequenz verlängerten TSH-Rezeptors

### 1. Insertion von Sequenzen für das FLAG-Epitop sowie eine 6His-Peptid-Sequenz in eine TSH-Rezeptor-CDNA-Sequenz

Eine vollständige humane TSH-Rezeptor-cDNA (Libert et al., Biochem. Biophys. Res. Commun. 165: 1250-1255, (1989)) wurde nach einer vorbeschrieben Technik im pSVL-Vektor subkloniert (pSVL-TSHR-Plasmid). P1- und P2-Primer wurden dazu verwendet, das von kommerziell erhältlichen Antikörpern erkannte FLAG-Octapeptid-Epitop (N-Asp Tyr Lys Asp Asp Asp Asp Lys-C; vgl. Prospekt IBI FLAG® SYSTEM der Fa. INTEGRA Biosciences) am C-Terminus des TSH-Rezeptors einzuführen. Das 0,5 kb Bgl II-Bam HI-Fragment der TSH-Rezeptor-cDNA wurde auf herkömmliche Weise durch PCR (Polymerase Chain Reaction) unter Verwendung von thermostabiler Taq-DNA-Polymerase vervielfältigt. Der P1-Primer enthält eine Bgl II-Restriktionsstelle, der P2-Primer enthält die FLAG-Epitop-Sequenz (unterstrichen), gefolgt von einem Stopcodon und einer Bam HI-Restriktionsstelle. Das PCR-Fragment wurde mit Bgl II und Bam HI geschnitten und dazu verwendet, das gleiche Fragment in der TSH-Rezeptor-cDNA in dem pSVL-TSH-Plasmid zu ersetzen. Das erhaltene Konstrukt erhielt die Bezeichnung pSVL-TSHR-FLAG.

P1- und P3-Primer wurden dann verwendet, um das 6His-Peptid direkt nach der FLAG-Epitop-Sequenz am C-Terminus des TSH-Rezeptors einzuführen. Das 0,5 kb Bgl II-Bam HI-Fragment der TSH-Rezeptor-cDNA in dem pSVL-TSHR-FLAG-Plasmid wurde nach der PCR-Technik vervielfältigt. Der P1-Primer enthält eine Bgl II-Restriktionsstelle, der P3-Primer enthält die Sequenz für das 6His-Peptid, gefolgt von einem Stopcodon und einer Bam HI-Restriktionsstelle. Das PCR-Fragment wurde mit Bgl II und Bam HI geschnitten und dazu verwendet, das entsprechende Fragment in der TSH-Rezeptor-CDNA im pSVL-TSHR-FLAG-Plasmid zu ersetzen. Der erhaltene Vektor erhielt die Bezeichnung pSVL-TSHR-FLAG-6His. Die Nukleotidsequenz der verlängerten cDNA wurde nach dem Sanger-Verfahren bestätigt.

### 2. Konstruktion eines pTM1-TSHR-FLAG-6His-Expressionsplasmids

Das TSHR-FLAG-6His-DNA-Segment wurde in den pTM1-Vektor (vgl. Moss et al., Nature, 348, S.19-92, (1990)) strangabwärts von der nicht translatierten Encephalomyocarditisvirus (EMCV)-Leadersequenz, die den Wirkungsgrad der mRNA-Translation erhöht, inseriert. Die Nco I-Schnittstelle im pTM1-Vektor enthält das Translations-Initiations-Codon, das für die Insertion des 5'-Endes der für das Protein codierenden cDNA genutzt werden sollte.

P4- und P5-Primer wurden dazu verwendet, eine Schnittstelle für die Rca I-Restriktase im Startcodon-Bereich der TSH-Rezeptor-cDNA im pSVL-TSHR-FLAG-Plasmid zu erzeugen. Diese Schnittstelle ist mit der Nco I-Restriktionsstelle im pTM1-Plasmid kompatibel. Das 0,7 kb Ava I-Afl III-Fragment der TSH-Rezeptor-cDNA wurde durch PCR vervielfältigt, wobei das pSVL-TSHR-FLAG-6His-Plasmid als Matrize diente. Der P4-Primer enthält Ava I-und Rca I-Restriktionsstellen, der P5-Primer enthält die Afl III-Restriktionsstelle. Das PCR-Fragment wurde mit Ava I und Afl III geschnitten und dazu verwendet, das gleiche Fragment in der TSH-Rezeptor-cDNA im pSVL-TSHR-FLAG-6His-Plasmid zu ersetzen. Aus diesem Plasmid wurde die verlängerte 2,3 kb TSH-Rezeptor-cDNA mit den Sequenzen für das FLAG-Epitop und das 6His-Peptid mit Rca I und Bam HI freigesetzt und im pTM1-Plasmid an den Nco I- und Bam HI-Schnittstellen strangabwärts von der EMCV-Leader-Sequenz subkloniert. Das erhaltene Konstrukt erhielt die Bezeichnung pTM1-TSHR-FLAG-6His. Die Nukleotidsequenz wurde nach dem Sanger-Verfahren bestätigt.

### 3. Konstruktion eines Vaccinia-Rekombinations-Plasmids p7.5K131-TSHR-FLAG-6His

Das pTM1-TSHR-FLAG-6His-Plasmid wurde durch Schneiden mit der Cla I-Restriktase linearisiert und zur Ausbildung stumpfer Enden mit Klenow-Fragment aufgefüllt. Nach einer Bam HI-Spaltung wurde das TSHR-FLAG-6His-DNA-Segment, das strangaufwärts von der untranslatierten EMCV-Leadersequenz flankiert wurde, isoliert und an Hind II- und Bam HI-Schnittstellen in den Vaccinia-Rekombinationsvektor p7.5K131 subkloniert. Das erhaltene Konstrukt erhielt die Bezeichnung p7.5K131-TSHR-FLAG-6His.

### 4. Erzeugung von Vacciniavirus-Rekombinanten

Die TSH-Rezeptor-cDNA unter Kontrolle des 7.5K-Early/Late-Promotors wurde durch homologe Rekombination in das Thymidinkinase (TK)-Gen des Genoms des Wildtyp-Vacciniavirus-Stamms Copenhagen inkorporiert (vgl. Kieny, M.P. et al., Nature 312, S.163-166, (1984)). Kurz gesagt, wurden zu diesem Zwecke konfluente CV1-Zellen mit dem temperaturempfindlichen Vacciniavirus ts7 infiziert und mit Vacciniavirus-DNA (Stamm Copenhagen) und dem p7.5K131-TSHR-FLAG-6His-Konstrukt nach der Calciumphosphat-Methode transfiziert. Die Viren wurden zwei Tage später geerntet, die TK-negativen Rekombinanten wurden aus dem Virusabkömmling in zwei Durchgängen einer Plaque-Reinigung über TK⁻ 143B-Zellen in Gegenwart von 0,1 mg/ml Bromdesoxyuridin selektiert. Rekombinante Virus-Plaques wurden isoliert, und positive Klone wurden nach Expression in infizierten HeLa-Zellen durch Western-Blot-Analyse auf TSH-Rezeptor identifiziert.

Die Proteine wurden dazu einer SDS-Gelelektrophorese auf einem 8% Auflösungs-Polyacrylamidgel (PAG) unterzogen (vgl. Laemmli, U.K., Nature, 227, S. 680-685 (1970)). Die aufgetrennten Proteine wurden auf eine Nitrozellulosemembran übertragen, unspezifische Bindungsstellen wurden innerhalb 1 h durch Inkubation mit TBS/5% Trockenmilch/0,02% Tween® 20-Lösung abgesättigt. Die Membran wurde über Nacht bei 4°C durch Umsetzung mit dem monoklonalen NCL-TSHR-Antikörper als Sonde für den TSH-Rezeptor behandelt, der 1:20 verdünnt war. Gebundene Antikörper wurden unter Verwendung des ECL® Western-Blot-Nachweis-Kits nachgewiesen.

Eine größere Vorratsmenge des rekombinanten Virus wurde in HeLa-Zellen hergestellt.

### 5. Expression des verlängerten TSH-Rezeptor-Polypeptids in HeLa-Zellen und Erzeugung von Zellfraktionen

HeLa-Monolayer-Zellen in 75 cm²-Schalen (etwa 15·10⁶ Zellen) wurden mit einer Plaque-bildenden Einheit (1 pfu) von rekombinantem Vacciniavirus pro Zelle infiziert und 24 h bei 37°C inkubiert. Infizierte Zellen wurden mit PBS gewaschen, geerntet und bei 1200 U/min pelletiert. Das erhaltene Zellpellet wurde in 0,3 ml eines Puffers resuspendiert, der 10 mM Tris-HCl, pH 7,6, 50 mM NaCl, 10% Glycerol sowie eine Proteaseinhibitoren-Mischung enthielt. Die Suspension wurde durch 20 Takte in einem Glas-Teflon-Homogenisator weiter homogenisiert und dann 15 min bei 800 g und 1 h bei 30.000 g zentrifugiert. Der Überstand (wasserlösliche Cytoplasmafraktion) wurde gesammelt. Das Membran-Pellet wurde mit 0,3 ml 1% Triton® X100 im gleichen Puffer extrahiert und bei 30.000 g 1 h ultrazentrifugiert. Der Überstand (Triton® X100-Membranextrakt) wurde gesammelt, das Pellet (unlösliches Protein) wurde in 0,3 ml eines SDS Elektrophoreseproben-Puffers resuspendiert. Die Menge des TSH-Rezeptors in 10 µl einer jeden Fraktion wurde durch Western-Blot-Analyse abgeschätzt.

Die Kinetik der TSH-Rezeptor-Produktion in HeLa-Zellen nach Infektion mit dem rekombinanten Vacciniavirus bei einer Multiplizität der Infektion von 1 ist in Fig. 1 gezeigt. Die Zellen wurden zu Zeitpunkten zwischen 0 und 24 h nach der Infektion gesammelt, durch SDS-Gelelektrophorese aufgetrennt und mit kommerziell erhältlichen monoklonalen NCL-TSHR-Antikörpern gegen den TSH-Rezeptor markiert. Das rekombinante Protein erschien 6 h nach der Infektion, und die Menge des Proteins erhöhte sich bis zu einem Zeitpunkt von 24 h nach der Infektion, wobei zu diesem Zeitpunkt die Zellen noch relativ gesund sind. Eine Inkubation für einen längeren Zeitraum führte zu einer Zellablösung und ihrem Tod. Eine Western-Blot-Analyse der infizierten Zellen zeigt verschiedene Formen des Rezeptors mit Molmassen von 230 bis 50 kDa, wobei die Hauptfraktion eine Molmasse von etwa 95 kDa aufwies. Das Ergebnis entspricht den aus der Literatur bekannten Daten (Ban T. et al., Endocrinology 131, S.815-829 (1992)). Im Falle der Infektion von HeLa-Zellen mit dem Wildtyp-Vacciniavirus war keinerlei Signal nachweisbar (Ergebnisse nicht gezeigt).

Die Verteilung des erzeugten TSH-Rezeptors auf unterschiedliche Fraktionen, die aus infizierten HeLa-Zellen erhalten wurden, ist in Fig. 3 gezeigt. In der wasserlöslichen Cytoplasma-Fraktion (Bande 2) ist nur eine geringe Menge des Rezeptors nachweisbar. Der Hauptteil des TSH-Rezeptors liegt in der Membran-Fraktion vor, aus der er mit Triton® X100 in wirksamer Weise extrahiert werden kann (Bande 3). Ein Teil des Rezeptors in der Membran-Fraktion wurde nur durch SDS solubilisiert (Bande 4). Dabei kann es sich um einen nicht extrahierbaren TSH-Rezeptor handeln, der fest in die Membranstruktur integriert ist.

Um zu testen, ob eine Abhängigkeit der Ausbeute des TSH-Rezeptors von der Intensität der Infektion besteht, wurde die TSH-bindende Aktivität von Zellen, die mit einer Infektions-Multiplizität von 1 sowie von 10 infiziert wurden, gemessen. Eine Erhöhung der Infektions-Multiplizität von 1 auf 10 erhöhte die Endausbeute des funktionalen TSH-Rezeptors bis zu 24 h nach der Infektion nicht.

### Bindungsassay für ¹²⁵I-TSH und Morbus Basedow-Autoantikörper

HeLa-Zellen in einer 75 cm²-Schale (etwa 15·10⁶ Zellen) wurden mit 1 bzw. 10 Plaque-bildenden Einheit(en) des rekombinanten Vacciniavirus pro Zelle infiziert und 24 h bei 37°C inkubiert. Infizierte Zellen wurden mit PBS gewaschen, geerntet und bei 1200 U/min pelletiert. Die erhaltenen Zellen wurden in 0,3 ml Puffer A (10 mM Tris-HCl, pH 7,6, 50 mM NaCl, 1% Triton® X100, 10% Glycerol), der mit Proteaseinhibitoren versetzt war, lysiert. Die Suspension wurde bei 30.000 g 1 h zentrifugiert, und der Überstand lieferte die TSH-Rezeptor-Präparation.

Der Assay wurde unter Verwendung von Komponenten des TRAK-Assay®-Kits in einem Gesamtvolumen von 100 µl durchgeführt, das sich aus 25 µl Lösung des rekombinanten TSH-Rezeptors (etwa 200 µg Gesamtprotein), 25 µl entweder Nullstandard-Serum oder Test-Serum sowie 50 µl einer ¹²⁵I-TSH-Lösung (20.000 cpm) zusammensetzte. Im Einklang mit den Herstelleranweisungen wurde 2 h inkubiert. Die Umsetzung wurde durch Zugabe von 1 ml eines Fällungsmittels gemäß Gebrauchsanweisung gestoppt. Die Röhrchen wurden bei 2000 g 10 min zentrifugiert, und der Überstand wurde entfernt. Die Gammastrahlung im Pellet wurde gemessen. Ergebnisse werden entweder ausgedrückt als gebundene Radioaktivität (in cpm - counts per minute) oder als Prozentsatz der Gesamtbindung, und zwar berechnet nach [(counts in Gegenwart des Test-Serums)/(counts in Gegenwart des Nullstandard-Serums)]·100%. Die unspezifische Bindung wurde in Gegenwart von 10⁻⁷ M TSH bestimmt und betrug etwa 10% der Gesamtbindung.

Der in den HeLa-Zellen erzeugte TSH-Rezeptor erwies sich im Hinblick auf der Bindung von TSH als voll funktionsfähig. Fig.2 zeigt die Bindung von ¹²⁵I-markiertem TSH mit Extrakte von infizierten HeLa-Zellen als Funktion der Zeit der Infektion. Es besteht ein klarer Zusammenhang zwischen der TSH-Rezeptor-Expression in HeLa-Zellen und der TSH-bindenden Aktivität der Zellextrakte (Fig. 1 und 2).

### Scatchard-Analyse der TSH-Bindung an infizierte HeLa-Zellen

HeLa-Zellen in Mikrotiterplatten mit 24 Vertiefungen (etwa 300.000 Zellen/Vertiefung) wurden mit einer Plaque-bildenden Einheit pro Zelle entweder des rekombinanten Vacciniavirus oder des Wildtyp-Vacciniavirus infiziert und 24 h bei 37°C inkubiert. Infizierte Zellen wurden 3 h bei 37°C in 0,2 ml modifiziertem Hank's-Puffer ohne NaCl inkubiert, wobei die Isotonie der Lösung durch 280 mM Saccharose, die mit 0,25% Kälberserumalbumin ergänzt war, gewährleistet wurde. Unterschiedliche Mengen von (a) ¹²⁵I-markiertem TSH oder (b) einer Mischung von ¹²⁵I-TSH (25.000 cpm) mit variierenden Konzentrationen von unmarkiertem TSH wurden diesem Puffer zugegeben. Am Ende der Inkubationsperiode wurden die Zellen schnell mit 1 ml des gleichen Puffers gewaschen und mit 1 ml 1N NaOH solubilisiert, und die Radioaktivität wurde in einem Gamma-Counter gemessen. Die spezifische TSH-Bindung wurde definiert als Unterschied zwischen der Gesamtbindung von ¹²⁵I-TSH in Abwesenheit sowie in Gegenwart von 10⁻⁷ M TSH. Die unspezifische Bindung betrug etwa 2% der Gesamtbindung. Die Dissoziationskonstante für die TSH-Bindung wurde nach dem Scatchard-Verfahren errechnet (Scatchard, G., Ann. N.Y. Acad. Sci. 51, 600-672, (1949)). Kontrolluntersuchungen betrafen die Bindung von ¹²⁵I-TSH an HeLa-Zellen, die mit dem Vacciniavirus vom Wildtyp infiziert waren.

Die Wechselwirkung von mit dem rekombinanten Virus infizierten HeLa-Zellen mit ¹²⁵I-markiertem TSH führte zu einer spezifischen und sättigbaren Bindung des Hormons. Die nach dem Scatchard-Verfahren erhaltenen Daten sind in Fig. 4 aufgetragen. Die Tatsache, daß die Scatchard-Kurve in Fig. 4 eine gerade Linie ist, weist auf die Existenz von nur einem Typ von Bindungsstellen hin. Aus der Steigung der Kurve kann eine Dissoziationskonstante von etwa 2·10⁻¹⁰ M errechnet werden. HeLa-Zellen, die mit dem Wildtyp-Vacciniavirus infiziert worden waren, zeigten keinerlei nachweisbare spezifische TSH-Bindung (Daten nicht gezeigt). Unter Zugrundelegung der Avogadro-Zahl errechnet sich eine Anzahl von etwa 150.000 hochaffinen TSH-Rezeptoren pro Zelle.

### Wechselwirkung des TSH-Rezeptors mit Morbus Basedow-Autoantikörpern

Der durch Expression des rekombinanten Vacciniavirus erhaltene rekombinante TSH-Rezeptor wurde auch auf seine Fähigkeit untersucht, in Seren von Patienten mit Morbus Basedow mit TSH-Rezeptor-Autoantikörpern zu wechselwirken. Die Anwesenheit derartiger Autoantikörper läßt sich anhand ihrer Fähigkeit nachweisen, die Bindung von ¹²⁵I-TSH an den TSH-Rezeptor zu inhibieren. Dabei ist die Menge der Autoantikörper in dem untersuchten Serum der gemessenen gebundenen Radioaktivität umgekehrt proportional. Mit dem TRAK-Assay®-Kit wurden Seren von sechs gesunden Individuen sowie von 13 Morbus Basedow-Patienten mit unterschiedlichen Autoantikörper-Spiegeln gemessen. Die Ergebnisse sind in Fig. 5 dargestellt. Sie zeigen, daß der durch Expression des rekombinanten Vacciniavirus in HeLa-Zellen erhaltene verlängerte TSH-Fusionsrezeptor von Morbus Basedow-Autoantikörpern spezifisch erkannt wird. So wurde bei den Autoantikörper-freien Kontrollseren eine 95-106%ige Bindung von ¹²⁵I-TSH erhalten, während bei der Messung von Seren von Morbus Basedow-Patienten nur eine 30-75%ige entsprechende Bindung erhalten wurde. Die Ergebnisse zeigten eine signifikante positive Korrelation zu den mit dem TRAK-Assay® Kit erhaltenen Werten.

### Intrazellulare cAMP-Messungen

HeLa Monolayer-Schichten in Mikrotiterplatten mit 24 Vertiefungen wurden mit einer Plaque-bildenden Einheit pro Zelle von entweder dem rekombinanten Vacciniavirus oder dem Wildtyp-Vacciniavirus infiziert und 24 h bei 37°C inkubiert. Infizierte Zellen wurde 1 h in modifiziertem Hank's-Puffer ohne NaCl (vgl. oben) inkubiert und dann eine weitere Stunde im gleichen Puffer, der 1mM Isobutylmethylxanthin sowie vorgegebene Konzentrationen an TSH enthielt. Zellulares cAMP wurde mit 65% Ethanol extrahiert und unter Verwendung des BIOTRAK® cAMP-Assay-Kits gemäß den Anweisungen des Herstellers gemessen. Kontrolluntersuchungen betrafen die Messungen der TSH-abhängigen cAMP-Produktion in HeLa-Zellen, die mit dem Wildtyp-Vacciniavirus infiziert waren. Es zeigte sich, daß die Einwirkung von TSH eine maximal ca. 6fache dosisabhängige Erhöhung der cAMP-Produktion bewirkte. Mit dem Wildtyp-Vacciniavirus infizierte HeLa-Zellen reagierten nicht. Somit erwies sich der rekombinante TSH-Fusionsrezeptor auch als funktional im Hinblick auf die Stimulation der intrazellulären cAMP-Produktion.

### Herstellung eines immobilisierten funktionalen rekombinanten TSH-Fusionsrezeptors

Mit dem rekombinanten Vacciniavirus infizierte HeLa-Zellen wurden nach einer 24 stündigen Inkubation bei 37°C wie weiter oben beschrieben geerntet. Das erhaltene Zellpellet (etwa 2·10⁸ Zellen) wurden anschließend in 3 ml Puffer (10 mM Tris pH 7,6; 50 mM NaCl; 1 % Triton® X-100; 10 % Glycerol) resuspendiert und bei -80°C eingefroren. Nach dem Auftauen wurde die solubilisierte Membranfraktion durch Zentrifugation (15 min bei 1000 g und 30 min bei 30.000 g) angereichert und von unlöslichen Bestandteilen abgetrennt.

Parallel dazu wurden 200 µl Ni-NTA-Agarose (bezogen von der Fa. Qiagen GmbH) mit 200 µl des gleichen Puffers versetzt und in eine kleine offene Säule gepackt.

Der von unlöslichen Bestandteilen befreite Zentrifugationsüberstand wurde auf die Säule aufgetragen und über Nacht bei 4°C rezirkuliert, um den TSH-Fusionsrezeptor über seinen terminalen 6His-Peptidrest an die Ni-NTA-Agarose zu koppeln.

Die beladene Ni-NTA-Agarose wurde anschließend mit 3 ml Vollblut abgesättigt (Hämoglobin bindet unspezifisch an Ni-NTA-Agarose), um die verbliebenen Ni-NTA-Bindungsstellen abzusättigen. Nach Entfernen des Säulenüberstands wurde die so behandelte Ni-NTA-Agarose mit dem beschriebenen Puffer auf ein Gesamtvolumen von 1,5 ml gebracht und in Portionen von 25 µl auf Eppendorf-Teströhrchen verteilt.

### Bestimmung von TSH-Rezeptor-Autoantikörpern unter Verwendung des immobilisierten TSH-Fusionsrezeptors

In die vorausgehend beschriebenen Eppendorf-Teströhrchen mit je 25 µl Ni-NTA-Agarose mit dem daran gebundenen TSH-Fusionsrezeptor wurden zu Meßzwecken jeweils 25 µl eines Serums bzw. 25 µl der Standardlösungen aus dem TRAK-Assay® zugegeben, mit dem Inhalt des Teströhrchens vermischt und anschließend 20 min bei Raumtemperatur unter ständigem Schütteln inkubiert. Anschließend wurden pro Teströhrchen 50 µl Tracer (¹²⁵-TSH aus dem TRAK-Assay®) zugegeben, und unter ständigem Schütteln wurde über Nacht bei 4°C weiter inkubiert.

Anschließend wurden die Röhrchen kurz bei Raumtemperatur zentrifugiert (1 min), 80 µl des Überstands wurden entfernt, und das Zentrifugationspellet wurde in 350 µl Puffer resuspendiert. Nach einer erneuten Zentrifugation wurden 320 µl des Überstands wiederum abpipettiert, und das erhaltene gewaschene Pellet wurde direkt in einem Gammacounter gemessen.

Bei der Messung der Standards aus dem TRAK-Assay® wurden die in Tabelle 1 wiedergegebenen Ergebnisse erhalten. Die zugehörige Standardkurve ist in Fig. 6 gezeigt.

Die Patientenseren wurden jeweils in Doppelbestimmungen gemessen, wobei aus den Einzelmeßwerten Mittelwerte gebildet wurden, die in Tabelle 2 eingegangen sind. Zum Vergleich wurden die gleichen Seren mit dem herkömmlichen TRAK-Assay® gemessen. Die erhaltenen Ergebnisse'sind ebenfalls in Tabelle 2 aufgeführt. Fig. 7 zeigt die Korrelation der nach dem herkömmlichen TRAK-Assay® erhaltenen Meßwerte zu den Meßwerten, die unter Verwendung des immobilisierten rekombinanten TSH-Fusionsrezeptors erhalten wurden.

Fig. 8 zeigt die Ergebnisse der Messungen von Seren von Normalpatienten und Morbus Basedow-Patienten in einer graphischen Darstellung. Die Gruppe der Morbus Basedow-Patienten läßt sich klar von der Gruppe der Normalpatienten unterscheiden.

Es zeigt sich somit, daß es erstmals gelungen ist, die Bestimmung von TSH-Rezeptor Autoantikörpern, wie sie für den Morbus Basedow charakteristisch sind, und unter Verwendung eines funktionalen immobilisierten TSH-Rezeptors durchzuführen.

**Tabelle 1**

| **Standardkurve unter Verwendung eines Ni-NTA-Agarose-gekoppelten rekombinanten TSH-Fusionsrezeptors** | | | |
|---|---|---|---|
| STANDARD (aus TRAK-Assay®) | Konzentration | CPM | B/B0 |
| S0 | 0 | 2103,1 | 100 |
| S1 | 5 | 1957,3 | 93,1 |
| S2 | 15 | 1727,6 | 82,1 |
| S3 | 45 | 1419,8 | 67,5 |
| S4 | 135 | 899,4 | 42,8 |
| S5 | 405 | 564,5 | 26,8 |

**Tabelle 2**

| **Vergleichende Bestimmung von TSH-Rezeptor-Autoantikörpern unter Verwendung des TRAK-Assay® sowie unter Verwendung des immobilisierten rekombinanten TSH-Fusionsrezeptors** | | | |
|---|---|---|---|
| **Morbus Basedow Patienten** | | **Kontrollgruppe** | |
| TRAK | rek. TSHr | TRAK | rek. TSHr |
| 176,5 | 772,3 | 5,1 | 5,3 |
| 149,3 | 1164,8 | 1,1 | 6,7 |
| 38,6 | 73,9 | 6,0 | 9,2 |
| 55,1 | 96,6 | 1,8 | 8,3 |
| 24,3 | 53,6 | 4,7 | 4,8 |
| 79,6 | 237,6 | 5,2 | 6,8 |
| 104,6 | 385,2 | 3,9 | 8,7 |
| 142,4 | 359,1 | 3,1 | 6,8 |
| 189,0 | 1529,5 | 3,6 | 5,2 |
| 360,7 | 1788,3 | 3,3 | 6,6 |
| 22,6 | 67,7 | 1,1 | 7,1 |
| 133,4 | 627,4 | 7,6 | 6,5 |
| 81,7 | 168,7 | 3,4 | 8,2 |
| 100,2 | 668,8 | 3,7 | 6,9 |
| 121,9 | 771,2 | 5,8 | 1,5 |
| 45,1 | 142,4 | 1,8 | 5,7 |
| 16,0 | 18,9 | 1,5 | 5,5 |
| 23,7 | 97,2 | 5,2 | 3,8 |
| | | 2,5 | 7,1 |
| | | 4,5 | 5,5 |
| | | 5,1 | 7,7 |
| | | 2,5 | 6,2 |
| | | 3,4 | 5,8 |
| | | 5,5 | 4,4 |

### B. Herstellung und Prüfung eines biotinylierten TSH-Fusionsrezeptors (TSHR-BIO)

### Herstellung eines um biotinylierte BCCP-Sequenzen verlängerten TSH-Rezeptorfragments

### 1. Anfügung von BCCP-Sequenzen an eine TSH-Rezeptor cDNA

Es wurde mit einem Plasmid pSK1 gearbeitet, das die Sequenz für die 87 Aminosäuren umfassende C-terminale Domäne der Biotin-Carboxyl-Carrier-Protein (BCCP) Untereinheit einer prokaryontischen Acetyl-CoA-Carboxylase, und zwar der aus E.coli, enthält. Diese Sequenz wurde auf an sich bekannte Weise (vgl. auch oben unter A.1.) nach der PCR-Technik unter Verwendung der Primer P10 (der eine Stelle für die Restriktase BstEII enthält) und P11 (der ein Stopcodon und eine Stelle für die Restriktase BamHI enthält) vervielfältigt, wobei ein BstEII/BamHI PCR-Fragment erhalten wurde. Das Plasmid p7,5K131-TSHR-FLAG-6HIS (vgl. oben A.3.) wurde mit den Restriktasen BstEII und BamHI geschnitten. Dabei wurde ein BstEII/BamHI-Fragment erhalten, das auch 39 Aminosäuren vom C-Terminus der cDNA des TSH-Rezeptors enthielt. Dieses BstEII/BamHI-Fragment wurde aus dem letztgenannten Plasmid entfernt und durch das o.g. BstEII/-BamHI-PCR-Fragment ersetzt, und es wurde auf diese Weise das neue Plasmid p7,5K131-TSHR-BIO erhalten.

Die relevante Nukleotidsequenz wurde nach dem Sanger-Verfahren bestätigt. Das erhaltene Plasmid enthält die Sequenz für die N-terminalen 725 Aminosäuren des humanen TSH-Rezeptors (ohne die eine C-terminale Teilsequenz des natürlichen Rezeptors, der in vollständiger Form 764 Aminosäuren umfaßt) in Verknüpfung mit der 87 Aminosäuren umfassenden C-terminalen Domäne der Biotin-Carboxyl-Carrier-Protein-Untereinheit der Acetyl-CoA-Carboxylase von E.coli.

### 2. Erzeugung von Vacciniavirus-Rekombinanten

Die TSHR-BIO cDNA unter der Kontrolle des p7,5 Early/Late-Promotors wurde in das Genom des Vacciniavirus-Stammes Copenhagen vom Wildtyp durch homologe Rekombination in das Thymidinkinase (TK) Gen eingefügt (Metzger et al, 1994). Kurz gesagt wurden konfluente CV1 Zellen mit dem temperaturempfindlichen Vaccinia-Virus ts7 infiziert und mit Vaccinia-Virus DNA und dem p7,5K131-TSHR-BIO-Konstrukt nach dem Calciumphosphat-Verfahren transfiziert. Die Viren wurden 2 Tage später geerntet, und die TK-negativen Rekombinanten wurden aus dem Stammvirus in zwei Durchgängen einer Plaque-Reinigung auf TK⁻143B-Zellen in Gegenwart von 0,1 mg/ml Bromdesoxyuridin selektiert. Rekombinante Virus-Plaques wurden isoliert, und positive Klone wurden wie oben unter A.4. durch Western Blot-Analyse auf den TSH-Rezeptor identifiziert. Die für die Bindungstests benötigten größeren Mengen des Virus wurden in HeLa-Zellen hergestellt.

### 3. Herstellung des TSH-Rezeptor-Extrakts

Konfluente HeLa-Zellen in einer 75 cm²-Platte wurden mit 1 pfu rekombinantem Vaccinia-Virus pro Zelle infiziert und 24 Stunden bei 37°C inkubiert. Infizierte Zellen wurden mit PBS gewaschen, geerntet und bei 1200 U/min pelletiert. Die erhaltenen Zellen wurden in 0,3 ml Puffer A (10 mM Tris-HCl, pH 7,6, 50 mM NaCl, 1 % Triton X100, 10 % Glycerol, Protease-Inhibitoren) einer Lyse durch Einfrieren/Auftauen und eine Inkubation für 30 Minuten bei 4°C unterzogen. Die Suspension wurde bei 30.000 g für 1 Stunde zentrifugiert, und der Überstand (etwa 8 mg/ml Gesamtprotein) lieferte die TSH-Rezeptor-Präparation.

### Bindungeassay für ¹²⁵I-TSH und Morbus Basedow-Autoantikörper

Unfraktionierte Seren von gesunden Individuen und Patienten mit Basedow-Schilddrüsenüberfuhktion verschiedenen Grades wurden zur Untersuchung der Bindung von Autoantikörpern an den biotinylierten TSH-Rezeptor verwendet. Der Bindungsassay wurde in einem Gesamtvolumen von 210 µl durchgeführt, das sich aus 50 µl TSH-Rezeptorextrakt, 50 µl entweder Nullstandardserum oder Testserum, 10 µl Streptavidin-Agarose im Gleichgewicht mit Puffer A und 100 µl ¹²⁵⁻TSH (etwa 20.000 cpm) zusammensetzte. Die Mischung wurde zwei Stunden bei Raumtemperatur unter anhaltendem Durchmischen inkubiert, mit 0,3 ml Puffer A unter Zentrifugation gewaschen, und die Gammastrahlung im Pellet wurde gezählt. Die unspezifische Bindung wurde in Gegenwart von 10⁻⁷ M TSH bestimmt, und sie betrug weniger als 10 % der Gesamtbindung.

Kontrollmessungen, die die Bindung von ¹²⁵I-TSH und von Patienten-Autoantikörpern an einen solubilisierten porcinen TSH-Rezeptor betrafen, wurden unter Verwendung des TRAK Assay® (Brahms Diagnostica GmbH) im Einklang mit den Anweisungen des Herstellers durchgeführt.

### Ergebnisse

Es wurde gezeigt, daß die verwendete C-terminale prokaryontische BCCP-Domäne eine effektive posttranslationale Biotinylierung des TSHR-Fusionsproteins, das ein funktionales Fragment des vollständigen natürlichen TSH-Rezeptors enthält, auch bei einer Expression in tierischen Zellen (HeLa-Zellen) gewährleistet. Der neue Fusionsrezeptor TSHR-BIO konnte unter Verwendung des Vaccinia-Virus-Expressionssystems exprimiert werden. HeLa-Zellen, die mit dem rekombinanten Virus infiziert wurden, erzeugten große Mengen des biotinylierten TSH-Rezeptors in einer Größenordnung von 120.000 Molekülen pro Zelle. Dieser Wert ist um ein bis zwei Größenordnungen größer als die Anzahl von TSH-Rezeptoren in Schilddrüsenzellen und ist vergleichbar mit der Rezeptorzahl der besten bisher bekannten stabil transfizierten Säugetierzellklone. Der unter Verwendung des rekombinanten Vacciniavirus hergestellte Fusionsrezeptor TSHR-BIO erwies sich als vollständig funktional, d.h. er band TSH mit einer Dissoziationskonstanten K_{d} von 2, 2 ± 0,1 x 10⁻¹⁰ M. Das exprimierte Protein war mit hoher Effizienz biotinyliert, und wenigstens 80 % des rekombinanten Rezeptors TSHR-BIO wurden an Streptavidin gebunden. Der an Streptavidin-Agarose immobilisierte TSHR-BIO-Rezeptor wurde zum Nachweis von die TSH-Bindung an den TSH-Rezeptor der Schilddrüse inhibierenden Immunglobulinen in unfraktionierten Seren verwendet. Es bestand eine hohe positive Korrelation zwischen den bei diesem Assay erhaltenen Ergebnissen und den mit dem kommerziell erhältlichen TRAK Assay® (Brahms Diagnostica GmbH) erhaltenen Ergebnissen, der mit einem solubilisierten porcinen TSH-Rezeptor arbeitet (r = 0,85; P < 0,0001, 62 Seren).

Die Ergebnisse zeigen erstmalig einen festphasengebundenen biotinylierten TSH-Rezeptor, der eine Teilsequenz des vollständigen humanen TSH-Rezeptors enthält und bezüglich der Bindung von TSH und von pathologischen Autoantikörpern voll funktional ist, so daß er für ein neues, praktisches Verfahren für die Diagnose von Autoimmun-Hyperthyreose geeignet ist.

## Patentansprüche

1. Rezeptorbindungsassay zur Bestimmung von TSH-Rezeptor-Autoantikörpern in einer biologischen Probe,
bei dem man die Probe in einer Reaktionsmischung gleichzeitig oder nacheinander mit einer TSH-Rezeptor-Präparation sowie wenigstens einem von (i) einem Kompetitor und (ii) einem Mittel für die Abtrennung des TSH-Rezeptors und der daran gebundenen Bestandteile der Reaktionsmischung von der flüssigen Phase umsetzt, wobei einer der genannten Bestandteile der Reaktionsmischung markiert oder selektiv markierbar ist, und bei dem man den durch Bindung der zu bestimmenden Autoantikörper und/oder des Kompetitors an die TSH-Rezeptor-Präparation gebildeten Komplex von der flüssigen Reaktionsmischung abtrennt und aus der Menge der Markierung in dem genannten Komplex auf die Anwesenheit und/oder Menge der zu bestimmenden Autoantikörper zurückrechnet,
**dadurch gekennzeichnet, daß** man als TSH-Rezeptor-Präparation eine Präparation eines rekombinanten TSH-Fusionsrezeptors verwendet, der gegenüber einem funktionalen TSH-Rezeptor um einen Peptidrest verlängert ist, der einen Peptidrestabschnitt enthält, der ausgewählt ist aus dem Markerpeptid N-Asp Tyr Lys Asp Asp Asp Asp Lys-C (FLAG-Oktapeptid), einem 6His-Peptid oder einer posttranslational biotinylierten Aminosäuresequenz, und daß der rekombinante TSH-Fusionsrezeptor mit Hilfe des genannten Peptidrestabschnitts selektiv markiert oder markierbar und/oder durch Bindung an einen geeigneten Bindungspartner immobilisiert oder immobilisierbar ist, ohne daß die für den Rezeptorbindungsassay erforderliche Funktionalität des TSH-Rezeptors signifikant beeinträchtigt ist.

2. Rezeptorbindungsassay nach Anspruch 1, **dadurch gekennzeichnet, daß** der Peptidrest eine Länge von bis zu 600 Aminosäuren, inbesondere von 6 bis 200 Aminosäuren, aufweist.

3. Rezeptorbindungsassay nach Anspruch 2, **dadurch gekennzeichnet, daß** der Peptidrest ein C-terminaler Peptidrest ist.

4. Rezeptorbindungsassay nach Anspruch 1, **dadurch gekennzeichnet, daß** der immobilisierte selektive Bindungspartner ein selektiver Antikörper gegen das Markerpeptid oder ein mit dem 6His-Peptid reagierendes Metallchelatharz ist.

5. Rezeptorbindungsassay nach Anspruch 4, **dadurch gekennzeichnet, daß** das Metallchelatharz Ni-NTA-Agarose ist.

6. Rezeptorbindungsassay nach Anspruch 1, **dadurch gekennzeichnet, daß** die posttranslational biotinylierte Aminosäuresequenz die 87 Aminosäuren umfassende C-terminale Domäne der Biotin-Carboxyl-Carrier-Protein-Untereinheit einer prokaryontischen Acetyl-COA-Carboxylase ist.

7. Rezeptorbindungsassay nach Anspruch 1 oder 6, **dadurch gekennzeichnet, daß** der selektive Bindungspartner Streptavidin-Agarose ist.

8. Rezeptorbindungsassay nach einem der Ansprüche 1 bis 7, **dadurch gekennzeichnet, daß** der selektive Bindungspartner an eine Mikrofestphase gebunden oder als Beschichtung eines Teströhrchens oder einer Mikrotiterplatte vorliegt.

9. Rezeptorbindungsassay nach einem der Ansprüche 1 bis 8, **dadurch gekennzeichnet, daß** der rekombinante TSH-Fusionsrezeptor durch Expression der cDNA eines funktionalen humanen TSH-Rezeptors, die um eine für den Peptidrest codierende DNA-Sequenz verlängert und in das Genom eines rekombinanten Vacciniavirus inseriert war, in mit dem rekombinanten Vacciniavirus infizierten Warmblüterzellen hergestellt wurde.

10. Rezeptorbindungsassay nach Anspruch 9, **dadurch gekennzeichnet, daß** die Warmblüterzellen HeLa-Zellen sind.

11. Rezeptorbindungsassay nach einem der Ansprüche 1 bis 10, **dadurch gekennzeichnet, daß** die zu bestimmenden Autoantikörper rezeptorstimulierende Autoantikörper sind, deren Auftreten in einem Humanserum für den Morbus Basedow charakteristisch ist.

12. Rekombinanter TSH-Fusionsrezeptor, der die Aminosäuresequenz eines funktionalen humanen TSH-Rezeptors aufweist, die am C-Terminus um einen Peptidrest verlängert ist, der das Markerpeptid N-Asp Tyr Lys Asp Asp Asp Asp Lys-C und/oder einen 6His-Peptidrest und/oder eine posttranslational biotinylierte Aminosäuresequenz enthält, ohne daß die Bindungsfähigkeit des TSH-Rezeptors gegenüber TSH oder TSH-Rezeptor-Autoantikörpern beeinträchtigt ist.

13. Rekombinanter TSH-Fusionsrezeptor nach Anspruch 12, **dadurch gekennzeichnet, daß** die posttranslational biotinylierte Aminosäuresequenz die 87 Aminosäuren umfassende C-terminale Domäne der Biotin-Carboxyl-Carrier-Protein-Untereinheit einer prokaryontischen Acetyl-CoA-Carboxylase ist.

14. Rekombinanter TSH-Fusionsrezeptor nach Anspruch 12, **dadurch gekennzeichnet, daß** er an seinem C-Terminus nur um das Markerpeptid und einen benachbarten endständigen 6His-Peptidrest verlängert ist.

15. Rekombinanter TSH-Fusionsrezeptor nach einem der Ansprüche 12 bis 14, **dadurch gekennzeichnet, daß** er durch Expression einer cDNA-Sequenz eines funktionalen humanen TSH-Rezeptors, die um eine für den Peptidrest codierende DNA-Sequenz verlängert ist und in das Genom eines rekombinanten Vacciniavirus inseriert ist, in mit dem rekombinanten Vacciniavirus infizierten Warmblüterzellen, insbesondere HeLa-Zellen, hergestellt wurde.

16. Vacciniavirus, in dessen Genom am Ort des Thymidinkinasegens eine DNA-Sequenz exprimierbar inseriert ist, die für den humanen TSH-Rezeptor sowie einen diesen verlängernden Peptidrest codiert, wobei die für den Peptidrest codierende DNA für das Markerpeptid N-Asp Tyr Lys Asp Asp Asp Asp Lys-C sowie einen C-terminalen 6His-Peptidrest oder für eine posttranslational biotinylierbare Biotin-Carboxyl-Carrier-Protein-Untereinheit eines prokaryontischen Biotin-Enzyms, insbesondere für die 87 Aminosäuren umfassende C-terminale Domäne der Biotin-Carboxyl-Carrier-Protein-Untereinheit der Acetyl-CoA-Carboxylase von E. coli, codiert.

17. Reagenziensatz zur Durchführung eines Rezeptorbindungsassays nach einem der Ansprüche 1 bis 11, **dadurch gekennzeichnet, daß** er, neben weiteren üblichen Komponenten eines entsprechenden Reagenziensatzes, Teströhrchen umfaßt, deren Wände beschichtet sind entweder mit (i) einem Metallchelatharz, an das über einen geeigneten Peptidrest ein funktionaler rekombinanter TSH-Fusionsrezeptor mit einem 6-His-Peptidrest gebunden ist, oder (ii) Streptavidin-Agarose, an die ein rekombinanter TSH-Fusions-Rezeptor mit einer posttranslational biotinylierten Aminosäuresequenz gebunden ist.

18. Reagenziensatz nach Anspruch 17, **dadurch gekennzeichnet, daß** das Metallchelatharz Ni-NTA-Agarose ist und der Peptidrest einen endständigen 6His-Peptidrest enthält.

## Claims

1. Receptor binding assay for the determination of TSH receptor autoantibodies in a biological sample, in which the sample is reacted in a reaction mixture simultaneously or in succession with a TSH receptor preparation and at least one of (i) a competitor and (ii) an agent for separating the TSH receptor and the components of the reaction mixture which are bound thereto from the liquid phase, one of said components of the reaction mixture being labelled or being capable of being selectively labelled, and in which the complex formed by binding the autoantibodies to be determined and/or the competitor to the TSH receptor preparation is separated from the liquid reaction mixture and the presence and/or amount of the autoantibodies to be determined are calculated from the amount of the marker in said complex,
**characterized in that** the TSH receptor preparation used is a preparation of a recombinant TSH fusion receptor which, compared with a functional TSH receptor, is lengthened by a peptide residue which contains a peptide residue segment which is selected from the marker peptide N-Asp Tyr Lys Asp Asp Asp Asp Lys-C (FLAG octapeptide), a 6His peptide or a posttranslationally biotinylated amino acid sequence, and that the recombinant TSH fusion receptor is selectively labelled or capable of being selectively labelled with the aid of said peptide residue segment and/or is immobilized or capable of being immobilized by binding to a suitable binding partner, without the TSH receptor functionality required for the receptor binding assay being significantly impaired.

2. Receptor binding assay according to Claim 1, **characterized in that** the peptide residue has a length of up to 600 amino acids, in particular from 6 to 200 amino acids.

3. Receptor binding assay according to Claim 2, **characterized in that** the peptide residue is a C-terminal peptide residue.

4. Receptor binding assay according to Claim 1, **characterized in that** the immobilized selective binding partner is a selective antibody against the marker peptide or a metal chelate resin reacting with the 6His peptide.

5. Receptor binding assay according to Claim 4, **characterized in that** the metal chelate resin is Ni-NTA-agarose.

6. Receptor binding assay according to Claim 1, **characterized in that** the posttranslationally biotinylated amino acid sequence is the C-terminal domain, comprising 87 amino acids, of the biotin carboxyl carrier protein subunit of a procaryotic acetyl-COA-carboxylase.

7. Receptor binding assay according to Claim 1 or 6, **characterized in that** the selective binding partner is streptavidin-agarose.

8. Receptor binding assay according to any of Claims 1 to 7, **characterized in that** the selective binding partner is present bound to a microsolid phase or as a coating of a test tube or of a microtitre plate.

9. Receptor binding assay according to any of Claims 1 to 8, **characterized in that** the recombinant TSH fusion receptor was prepared by expression of the cDNA of a functional human TSH receptor, which was lengthened by a DNA sequence coding for the peptide residue and was inserted into the genome of a recombinant vaccinia virus, in warm-blooded animal cells infected with the recombinant vaccinia virus.

10. Receptor binding assay according to Claim 9, **characterized in that** the warm-blooded animal cells are HeLa cells.

11. Receptor binding assay according to any of Claims 1 to 10, **characterized in that** the autoantibodies to be determined are receptor-stimulating autoantibodies whose occurrence in a human serum is characteristic of Graves' disease.

12. Recombinant TSH fusion receptor which has the amino acid sequence of a functional human TSH receptor which is lengthened at the C-terminus by a peptide residue which contains the marker peptide N-Asp Tyr Lys Asp Asp Asp Asp Lys-C and/or a 6His peptide residue and/or a posttranslationally biotinylated amino acid sequence, without the TSH receptor binding capability with respect to TSH or TSH receptor autoantibodies being impaired.

13. Recombinant TSH fusion receptor according to Claim 12, **characterized in that** the posttranslationally biotinylated amino acid sequence is the C-terminal domain, comprising 87 amino acids, of the biotin carboxyl carrier protein subunit of a procaryotic acetyl-COA-carboxylase.

14. Recombinant TSH fusion receptor according to Claim 12, **characterized in that** it is lengthened at its C-terminus only by the marker peptide and a neighbouring terminal 6His peptide residue.

15. Recombinant TSH fusion receptor according to any of Claims 12 to 14, **characterized in that** it was prepared by expression of a cDNA sequence of a functional human TSH receptor, which is lengthened by a DNA sequence coding for the peptide residue and is inserted into the genome of a recombinant vaccinia virus, in warm-blooded animal cells, in particular HeLa cells, infected with the recombinant vaccinia virus.

16. Vaccinia virus into whose genome a DNA sequence which codes for the human TSH receptor and a peptide residue lengthening said receptor is expressably inserted at the site of the thymidine kinase gene, the DNA coding for the peptide residue coding for the marker peptide N-Asp Tyr Lys Asp Asp Asp Asp Lys-C and a C-terminal 6His peptide residue or for a posttranslationally biotinylatable biotin carboxyl carrier protein subunit of a procaryotic biotin enzyme, in particular for the C-terminal domain, comprising 87 amino acids, of the biotin carboxyl carrier protein subunit of the acetyl-COA-carboxylase of E. coli.

17. Kit for carrying out a receptor binding assay according to any of Claims 1 to 11, **characterized in that**, in addition to further customary components of a corresponding kit, it comprises test tubes whose walls are coated either with (i) a metal chelate resin to which a functional recombinant TSH fusion receptor having a 6-His peptide residue is bound via a suitable peptide residue, or (ii) streptavidin-agarose to which a recombinant TSH fusion receptor having a posttranslationally biotinylated amino acid sequence is bound.

18. Kit according to Claim 17, **characterized in that** the metal chelate resin is Ni-NTA-agarose and the peptide residue contains a terminal 6His peptide residue.

## Revendications

1. Test de liaison à des récepteurs pour le dosage d'auto-anticorps contre les récepteurs de TSH dans un échantillon biologique,
dans lequel on fait réagir l'échantillon dans un mélange réactionnel simultanément ou successivement avec une préparation de récepteur de TSH ainsi qu'avec au moins une substance choisie entre (i) un compétiteur et (ii) un agent destiné à séparer de la phase liquide le récepteur de TSH et les composants du mélange réactionnel qui y sont liés, l'un des composants indiqués du mélange réactionnel étant marqué ou pouvant être marqué sélectivement, et dans lequel on sépare du mélange réactionnel liquide le complexe formé par liaison des auto-anticorps à doser et/ou du compétiteur à la préparation de récepteur de TSH, et à partir du degré de marquage dans le complexe mentionné, on le rapporte à la présence et/ou à la quantité d'auto-anticorps à doser,
**caractérisé en ce qu'**on utilise comme préparation de récepteur de TSH une préparation d'un récepteur de fusion de TSH recombiné, qui est prolongée par rapport à un récepteur de TSH fonctionnel d'un reste peptidique qui contient un segment choisi entre le peptide marqueur N-Asp Tyr Lys Asp Asp Asp Asp Lys-C (Oktapeptide-FLAG), un peptide 6His ou une séquence post-translationnelle d'amino-acides biotinylée, et **en ce que** le récepteur de fusion de TSH recombiné est sélectivement marqué ou marquable à l'aide du segment de reste peptidique mentionné et/ou immobilisé ou immobilisable par liaison à un partenaire de liaison approprié, sans ce que la fonctionnalité du récepteur de TSH nécessaire pour le test de liaison au récepteur soit altérée dans une mesure significative.

2. Test de liaison à des récepteurs suivant la revendication 1, **caractérisé en ce que** le reste peptidique présente une longueur allant jusqu'à 600 amino-acides, notamment de 6 à 200 amino-acides.

3. Test de liaison à des récepteurs suivant la revendication 2, **caractérisé en ce que** le reste peptidique est un reste peptidique à extrémité terminale C.

4. Test de liaison à des récepteurs suivant la revendication 1, **caractérisé en ce que** le partenaire de liaison sélectif immobilisé est un anticorps sélectif contre le peptide marqueur ou une résine de chélate métallique réagissant avec le peptide 6His.

5. Test de liaison à des récepteurs suivant la revendication 4, **caractérisé en ce que** la résine de chélate métallique consiste en Ni-NTA-Agarose.

6. Test de liaison à des récepteurs suivant la revendication 1, **caractérisé en ce que** la séquence post-translationnelle d'amino-acides biotinylée est le domaine à extrémité terminale C comprenant 87 amino-acides de la sous-unité biotine-carboxyl-support-protéine d'une acétyl-CoA-carboxylase procaryotique.

7. Test de liaison à des récepteurs suivant la revendication 1 ou 6, **caractérisé en ce que** le partenaire de liaison sélectif consiste en streptavidine-agarose.

8. Test de liaison à des récepteurs suivant l'une des revendications 1 à 7, **caractérisé en ce que** le partenaire de liaison sélectif est lié à une microphase solide ou est présent comme revêtement d'un tube à essai ou d'une plaque de micro-titrage.

9. Test de liaison à des récepteurs suivant l'une des revendications 1 à 8, **caractérisé en ce que** le récepteur de fusion de TSH recombiné a été produit par expression de l'ADNc d'un récepteur de TSH humain fonctionnel, qui avait été prolongé d'une séquence d'ADN codant le reste peptidique et qui avait été inséré dans le génome d'un virus vaccinal recombiné, dans des cellules d'animaux à sang chaud infectées par le virus vaccinal recombiné.

10. Test de liaison à des récepteurs suivant la revendication 9, **caractérisé en ce que** les cellules d'animaux à sang chaud sont des cellules HeLa.

11. Test de liaison à des récepteurs suivant l'une des revendications 1 à 10, **caractérisé en ce que** les auto-anticorps à doser sont des auto-anticorps stimulant des récepteurs, dont l'apparition dans un sérum humain est caractéristique de la maladie de Basedow.

12. Récepteur de fusion de TSH recombiné, qui présente une séquence d'amino-acides d'un récepteur de TSH humain fonctionnel qui est prolongé à l'extrémité terminale C de la longueur d'un reste peptidique qui contient le peptide marqueur N-Asp Tyr Lys Asp Asp Asp Asp Lys-C et/ou un reste peptidique 6His et/ou une séquence post-translationnelle d'amino-acides biotinylée, sans que la capacité de liaison du récepteur de TSH vis-à-vis de l'hormone TSH ou d'auto-anticorps contre les récepteurs de TSH soit altérée.

13. Récepteur de fusion de TSH recombiné suivant la revendication 12, **caractérisé en ce que** la séquence post-translationnelle d'amino-acides biotinylée est le domaine à extrémité terminale C comprenant 87 amino-acides de la sous-unité biotine-carboxyl-support-protéine d'une acétyl-CoA-carboxylase procaryotique.

14. Récepteur de fusion de TSH recombiné suivant la revendication 12, **caractérisé en ce qu'**il n'est prolongé, à son extrémité terminale C, que du peptide marqueur et d'un reste peptidique 6His terminal voisin.

15. Récepteur de fusion de TSH recombiné suivant l'une des revendications 12 à 14, **caractérisé en ce qu'**il a été produit par expression d'une séquence d'ADNc d'un récepteur de TSH humain fonctionnel, qui est prolongé d'une séquence d'ADN codant le reste peptidique et qui est inséré dans le génome d'un virus vaccinal recombiné, dans des cellules d'animaux à sang chaud infectées par le virus vaccinal recombiné, en particulier des cellules HeLa.

16. Virus vaccinal, dans le génome duquel est insérée au niveau du gène de la thymidine-kinase, d'une manière pouvant être exprimée, une séquence d'ADN qui code le récepteur de TSH humain ainsi qu'un reste peptidique prolongeant ce récepteur, l'ADN qui code le reste peptidique codant le peptide marqueur N-Asp Tyr Lys Asp Asp Asp Asp Lys-C ainsi qu'un reste de peptide 6His à extrémité terminale C ou une sous-unité biotine-carboxyl-support-protéine post-translationnelle pouvant être biotinylée, d'un enzyme procaryotique formé de biotine, en particulier le domaine à extrémité terminale C comprenant 87 amino-acides de la sous-unité biotine-carboxyl-support-protéine de l'acétyl-COA-carboxylase de E. coli.

17. Ensemble de réactifs pour la conduite de test de liaison à des récepteurs suivant l'une des revendications 1 à 11, **caractérisé en ce qu'**il comprend, à côté des autres composants classiques d'un ensemble de réactifs correspondant, des tubes à essai dont les parois sont tapissées de l'un ou l'autre (i) d'une résine de chélate métallique à laquelle est lié par l'intermédiaire d'un reste peptidique approprié, un récepteur de fusion de TSH recombiné fonctionnel portant un reste peptidique 6-His, ou (ii) de streptavidine-agarose auquel est lié un récepteur de fusion de TSH recombiné à séquence d'amino-acides post-translationnelle biotinylée.

18. Ensemble de réactifs suivant la revendication 17, **caractérisé en ce que** la résine de chélate métallique est le Ni-NTA-agarose et le reste peptidique est un reste de 6His terminal.
